# EUROPEAN PATENT APPLICATION

(11) **EP 2 612 690 A1**
(43) Date of publication of application: **10.07.2013**
(21) Application number: 11821368.5
(22) Date of filing: 19.04.2011
(51) Int. Cl.: A61M 27/00, A61B 1/00, A61M 25/00

(54) **DRAINAGE TUBE AND ENDOSCOPE WITH DRAINAGE TUBE**

(30) Priority: 30.08.2010 JP 2010192984
(71) Applicant: FUJIFILM Corporation, Tokyo 106-0031 (JP)
(72) Inventor: ITOH, Koji, Ashigarakami-gun (JP); IWASAKA, Masayuki, Ashigarakami-gun (JP)
(74) Representative: Klunker . Schmitt-Nilson . Hirsch
(86) International application number: PCT/JP2011/059646
(87) International publication number: WO 2012/029350

(57) **Abstract**

To provide a drainage tube and a drainage tube-including endoscope in which the drainage tube can be indwelled in a bile duct or pancreatic duct easily without forcing a patient to bear an excessive strain.

A drainage tube 200 is attached along the longitudinal direction of an endoscope insertion portion 15 to a front end portion of the endoscope insertion portion inserted into the bile duct 65 or pancreatic duct 66. The drainage tube 200 has: a front end side tube region 51 which is fixed to the outer circumference of the front end portion of the endoscope insertion portion 15 and which has radial elasticity so as not to collapse at the time of insertion into a duodenal papilla; and a rear end side tube region 53 which is provided so as to be consecutive to the front end side tube region 51, which has an extension length so as to be separate from the front end portion of the endoscope insertion portion 15 and which has lower radial elasticity than that of the front end side tube region 51.

## Description

### Technical Field

The present invention relates to a drainage tube and a drainage tube-including endoscope inserted into a bile duct or pancreatic duct opened to the duodenum.

### Background Art

A pancreatic drainage tube inserted into the pancreatic duct for drainage has been proposed (see Patent Literature 1). When one end side of the pancreatic drainage tube is inserted into the pancreatic duct, body fluid in the pancreatic duct can be drained to the duodenum.

### Citation List

### Patent Literature

Patent Literature 1: JP-A-2002-17868

### SUMMARY OF INVENTION

### Technical Problem

However, in order to indwell the drainage tube after insertion of a front end of an endoscope insertion portion into the bile duct or pancreatic duct, it is necessary to further insert another endoscope additionally. For this reason, an excessive strain is imposed on a patient to make the indwelling work itself difficult.

Moreover, when an endoscope is to be inserted after a drainage tube is indwelled, the drainage tube becomes an obstacle to insertion of the endoscope into the bile duct or pancreatic duct.

Therefore, an object of the invention is to provide a drainage tube and a drainage tube-including endoscope in which the drainage tube can be indwelled in a bile duct or pancreatic duct easily without forcing a patient to bear an excessive strain.

### Solution to Problem

The invention has the following configuration.
(1) It is a drainage tube attached along a longitudinal direction of an endoscope insertion portion to a front end portion of the endoscope insertion portion inserted into a bile duct or pancreatic duct, the drainage tube comprising: a front end side tube region which is fixed to an outer circumference of the front end portion of the endoscope insertion portion and which has radial elasticity so as not to collapse at the time of insertion into a duodenal papilla; and a rear end side tube region which is provided so as to be consecutive to the front end side tube region, which has an extension length so as to be separate from the front end portion of the endoscope insertion portion and which has lower radial elasticity than that of the front end side tube region.
(2) It is a drainage tube-including endoscope, in which the drainage tube is detachably fixed to the front end portion of the endoscope insertion portion.

### Advantageous Effects of Invention

According to the drainage tube and the drainage tube-including endoscope according to the invention, the drainage tube can be indwelled in a bile duct or pancreatic duct easily without forcing a patient to bear an excessive strain.

### BRIEF DESCRIPTION OF DRAWINGS

Fig. 1 is a view showing the external appearance of a drainage tube-including endoscope for the purpose of explaining an embodiment of the invention.
Fig. 2 is an enlarged perspective view of an endoscope front end portion shown in Fig. 1.
Fig. 3 is a side view schematically showing a drainage tube.
Fig. 4 is an enlarged perspective view of a front end side tube region.
Fig. 5A is a side view in which a tube tip of the front end side tube region is disposed in the same position in a tube axial direction as a front end surface of the endoscope front end portion, and Fig. 5B is a side view in which the tube tip of the front end side tube region protrudes outward from the front end surface of the endoscope front end portion.
Fig. 6 is a sectional view of the endoscope front end portion taken in a plane orthogonal to an axis.
Fig. 7 is a front view of the vicinity of the duodenum and a partly cutaway enlarged view of important part thereof.
Fig. 8 is an enlarged view of the endoscope front end portion and the drainage tube inserted into the bile duct at the time of drainage.

### DESCRIPTION OF EMBODIMENTS

An embodiment of the invention will be described below with reference to the drawings.

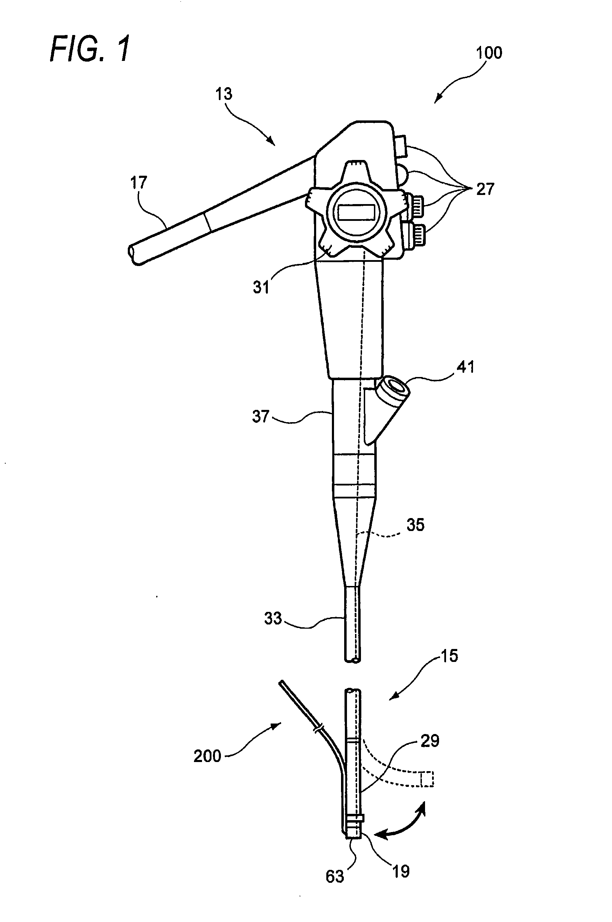

Fig. 1 is a view showing the external appearance of a drainage tube-including endoscope for the purpose of explaining an embodiment of the invention.

A drainage tube-including endoscope 100 has a main unit operation portion 13, and an endoscope insertion portion 15 which is provided so as to be consecutive to the main unit operation portion 13 and which is inserted into a body cavity (especially, into a bile duct or pancreatic duct). A universal cord 17 including various conduit lines and signal cables is connected to the main unit operation portion 13. A connector which can be detachably connected to a controller not shown is attached to a front end of the universal cord 17. Light emitted from a light source portion of the controller is fed to the drainage tube-including endoscope 100 through the connector and the universal cord 17 and guided as illumination light to an illumination optical system provided in an endoscope front end portion 19 of the endoscope insertion portion 15.

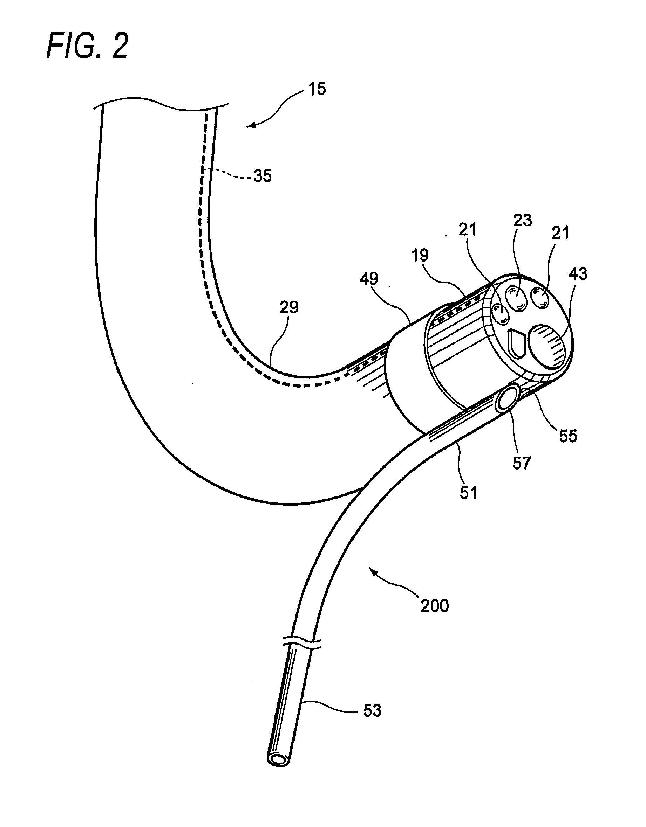

Fig. 2 is an enlarged perspective view of the endoscope front end portion 19 shown in Fig. 1.

An image pickup optical system 23 provided in the endoscope front end portion 19 has a not-shown image pickup device for picking up an image of an observation region illuminated by the illumination optical system 21. An image pickup signal of an observation image obtained from the image pickup device is outputted to the controller. A processor portion of the controller displays image information of the image-processed input image pickup signal on a not-shown display portion. In the series of processes, an instruction can be inputted from a keyboard or the like connected to the controller. A CCD image sensor or a CMOS image sensor is used as the image pickup device of the image pickup optical system 23.

As shown in Fig. 1, the main unit operation portion 13 has various buttons 27 such as an air feed/water feed button, a suction button, a shutter button, and a function changeover button which are provided side by side, and an angle knob 31 operated to curve a curvature portion 29 provided on a front end side of the endoscope insertion portion 15.

The endoscope insertion portion 15 has a soft portion 33, the curvature portion 29 and the endoscope front end portion 19 arranged in order viewed from the main unit operation portion 13 side. The soft portion 33 has flexibility and is provided so as to be consecutive to a base end side of the curvature portion 29. The curvature portion 29 is curved in such a manner that the angle knob 31 of the main unit operation portion 13 is operated and turned to pull a wire 35 which is provided so as to be inserted into the endoscope insertion portion 15. In this manner, the endoscope front end portion 19 can be turned to a desired direction.

A forceps insertion portion 41 in which a treatment tool such as forceps or a water feed device is inserted is provided in a connecting portion 37 between the main unit operation portion 13 and the endoscope insertion portion 15. The treatment tool inserted from the forceps insertion portion 41 is led out of a forceps port 43 (see Fig. 2) of the endoscope front end portion 19.

A drainage tube 200 is fixed along the longitudinal direction of the endoscope insertion portion on the outer circumference of the endoscope front end portion 19 (to be inserted into a body cavity) of the drainage tube-including endoscope 100. For example, the drainage tube 200 can be made of a material such as polyethylene, fluorocarbon resin, silicone or polyurethane. The drainage tube 200 may contain barium sulfate as a radiopaque component.

An attachment band 49, for example, made of an elastic material such as silicone rubber, is fixed to the drainage tube 200. The attachment band 49 is fastened to the outer circumference of the endoscope front end portion 19 by elastic force. Accordingly, the drainage tube 200 is detachably fixed to the endoscope front end portion 19. Because the drainage tube 200 is provided detachably, the drainage tube 200 can be afterward attached to an existing endoscope, and the drainage tube 200 can be exchanged for a new drainage tube 200 or a drainage tube 200 with a different length. Incidentally, the attachment band 49 may be fixed to the outer circumference of the endoscope front end portion 19 by an adhesive agent. In this case, the drainage tube 200 can be exchanged after the attachment band 49 is cut off.

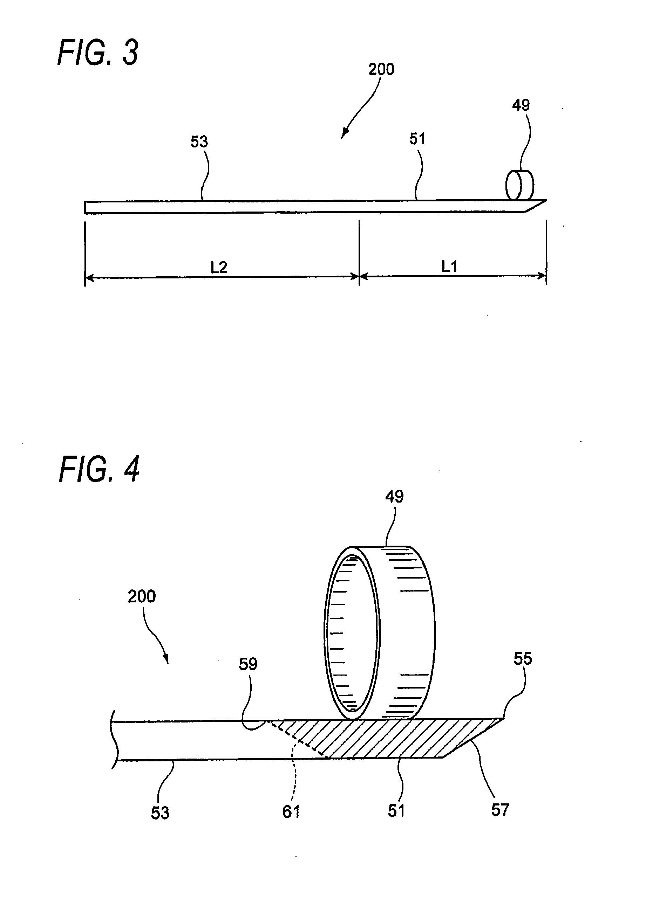

Fig. 3 is a side view schematically showing the drainage tube.

The drainage tube 200 has: a front end side tube region 51 which has a length L1, which is fixed to the outer circumference of the endoscope front end portion 19 and which has radial elasticity (elastic constant) so as not to collapse at the time of insertion into a duodenal papilla; and a rear end side tube region 53 which is provided so as to be consecutive to the front end side tube region 51, which has a length L2 (L1<L2) of an extension length so as to be separate from the endoscope front end portion 19 and which has lower radial elasticity than that of the front end side tube region 51.

Fig. 4 is an enlarged perspective view of the front end side tube region 51.

In the front end side tube region 51, the front end side of the tube is cut obliquely. When the front end side tube region 51 is fixed to the endoscope front end portion 19, a tube tip 55 which is an obliquely cut tip portion is disposed on the outer circumferential side of the endoscope front end portion 19. A front inclined surface 57 is provided as an inclined surface which gradually comes closer to the outer circumferential surface of the endoscope front end portion 19 as the position goes toward the front in the tube axial direction (toward the endoscope front end side). Because the drainage tube 200 tapered off in this manner is unitedly fixed to the endoscope front end portion 19, easiness of insertion of the endoscope front end portion 19 into a body cavity is improved. Moreover, because the front end of the front end side tube region 51 can be provided as a large opening, the fluid flow volume in the tube can be gained.

The front end side tube region 51 and the rear end side tube region 53 are connected to each other by an interface inclined wit respect to the tube axial direction. In detail, when the front end side tube region 51 is fixed to the endoscope front end portion 19, the rear end of the front end side tube region 51 has a rear inclined surface 61 which gradually comes closer to the outer circumference of the endoscope front end portion 19 as the position goes toward the rear. An interface tip 59 which is a tip of the rear inclined surface 61 is disposed on the outer circumferential side of the endoscope front end portion 19. With this configuration, when the endoscope front end portion 19 to which the drainage tube 200 is unitedly fixed is pulled out of the body cavity, the front end side tube region 51 is disposed along the endoscope front end portion 19 so that the interface tip 59 at the rear end of the front end side tube region is prevented from being pressed against the inner wall of the body cavity unexpectedly. Accordingly, the inner wall of the body cavity can be prevented from being damaged, so that easiness of extraction of the endoscope from the body cavity is improved.

Such a front end side tube region 51 having the front inclined surface 57 and the rear inclined surface 61 is made of a material having high elasticity. The drainage tube 200 is formed in such a manner that the rear end side tube region 53 made of another material having lower elasticity than that of the front end side tube region 51 is connected to the front end side tube region 51 by two-color molding, welding, adhesive bonding, etc. Moreover, the drainage tube 200 may be formed as a double pipe structure in which a tube body serving as the front end side tube region 51 is inserted and fitted into the front end of the rear end side tube region 53. In addition, the drainage tube 200 may be formed in such a manner that elasticity of only the front end side tube region 51 is increased by a curable resin of the same material whose curing is accelerated by light with a constant wavelength. That is, any structure and any production method may be used as long as the front end side tube region 51 can have higher elasticity than that of the rear end side tube region 53.

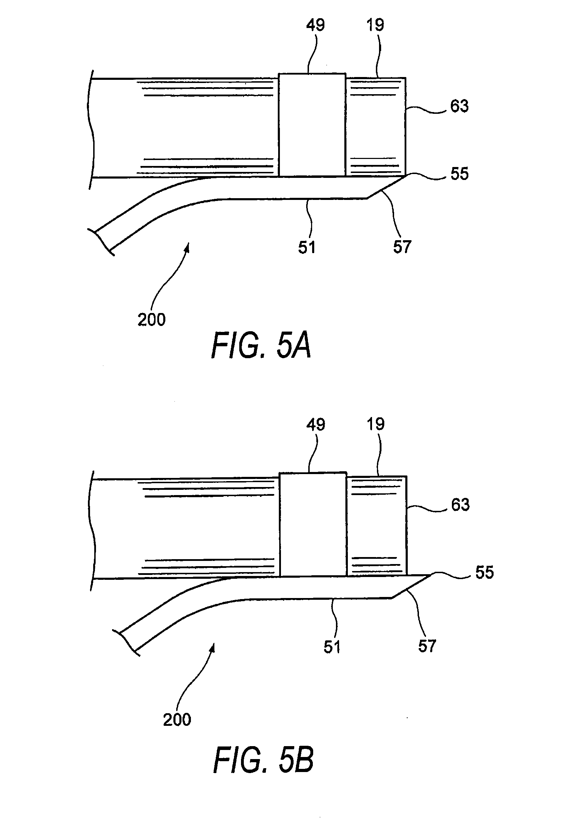

Fig. 5A is a side view in which the tube tip 55 of the front end side tube region 51 is disposed in the same position in the tube axial direction as a front end surface 63 of the endoscope front end portion 19. Fig. 5B is a side view in which the tube tip 55 of the front end side tube region 51 protrudes outward from the front end surface 63 of the endoscope front end portion 19.

As shown in Fig. 5A, the tube tip 55 of the front end side tube region 51 can be disposed in the same position in the tube axial direction as the front end surface 63 of the endoscope front end portion 19 or so as to be backed from the front end surface 63 (see Fig. 2). Because the tube tip 55 is disposed on the same plane with the front end surface 63 of the endoscope front end portion 19 or so as to be backed from the front end surface 63, the drainage tube 200 is prevented from coming into sight so that an observation image having a wide angle of view can be obtained. Moreover, when the tube tip 55 is backed from the front end surface 63 of the endoscope front end portion 19, transparent fluid which has just been supplied from the endoscope front end portion 19 is not discharged from the front end side tube region 51 immediately so that the supplied transparent fluid is stirred with the bile surely in front of the endoscope front end portion 19. In this manner, the bile can be stirred with the transparent fluid quickly so that transparency of a liquid in the periphery of the endoscope front end portion 19 can be improved to keep the field of view.

As shown in Fig. 5B, the tube tip 55 of the front end side tube region 51 can be disposed so as to protrude forward from the front end surface 63 of the endoscope front end portion 19. Because the tube tip 55 is disposed so as to protrude forward from the front end surface 63, the opening of the drainage tube 200 is prevented from abutting on the inner wall of the body cavity so that the bile can be discharged more smoothly without reduction in the discharged fluid flow volume. Accordingly, perfusion is apt to be formed so that the visual field of the endoscope can be kept quickly for sharp observation of the inside of the bile duct or pancreatic duct.

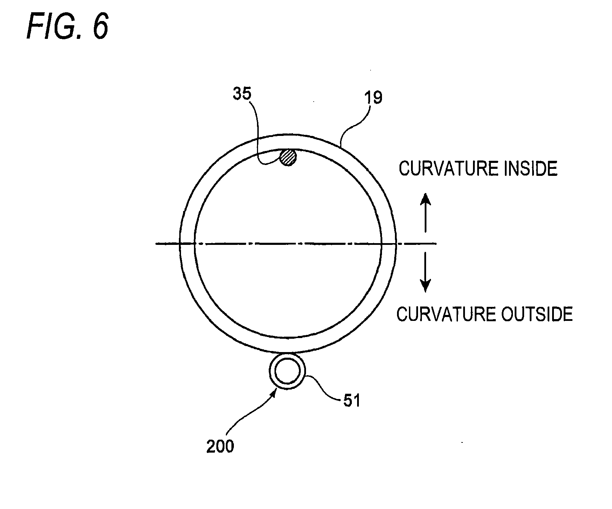

Fig. 6 is a sectional view of the endoscope front end portion 19 taken in a plane orthogonal to the axis.

The drainage tube-including endoscope 100 can be operated so that the curvature portion 29 of the endoscope insertion portion 15 is curved in only one direction by one wire 35. The rear side of the paper plane in Fig. 6 shows a rear portion in the direction of insertion. When the wire 35 is pulled by operation of the angle knob 31, the endoscope front end portion 19 is turned to the upside of Fig. 6 by the curvature of the curvature portion 29. Accordingly, the endoscope front end portion 19 shown in Fig. 6 has its upside provided as the curvature inside, and its downside provided as the curvature outside. Because the front end side tube region 51 is fixed to the endoscope front end portion 19 on the curvature outside, the drainage tube 200 is disposed on the outside of the curved curvature portion 29 so that the drainage tube 200 does not obstruct the operation of curving the curvature portion 29 when the curvature portion 29 is operated to be curved. Moreover, the rear end side tube region 53 of the drainage tube 200 does not collapse in the radial direction of the tube due to the curvature of the curvature portion 29.

Incidentally, the rear end side tube region 53 may have a length led out of the body from the position of the endoscope front end portion 19 inserted into the body cavity. In this manner, the base end side of the drainage tube 200 can be led out of the body cavity, and a suction pump, a collection vessel or a syringe which is not shown can be connected to the end of the base end side of the drainage tube 200 in order to collect the bile or the like. By connecting a suction pump, a collection vessel or a syringe, the bile or the like can be collected surely even when it cannot be discharged naturally by the long and narrow drainage tube 200.

When a suction pump is used, it is preferable that the suction pump performs suction based on pulsation. That is, when suction is not performed under a constant negative pressure but suction pressure is changed cyclically, fluidity of fluid in the tube is improved. In this manner, efficiency in collection of the bile or the like can be improved.

Operation of the drainage tube 200 and the drainage tube-including endoscope 100 configured as described above will be described below.

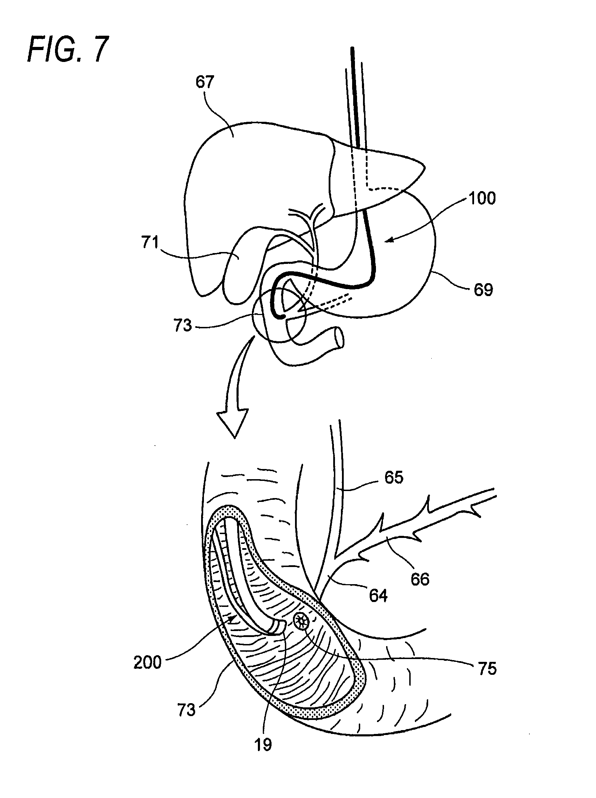

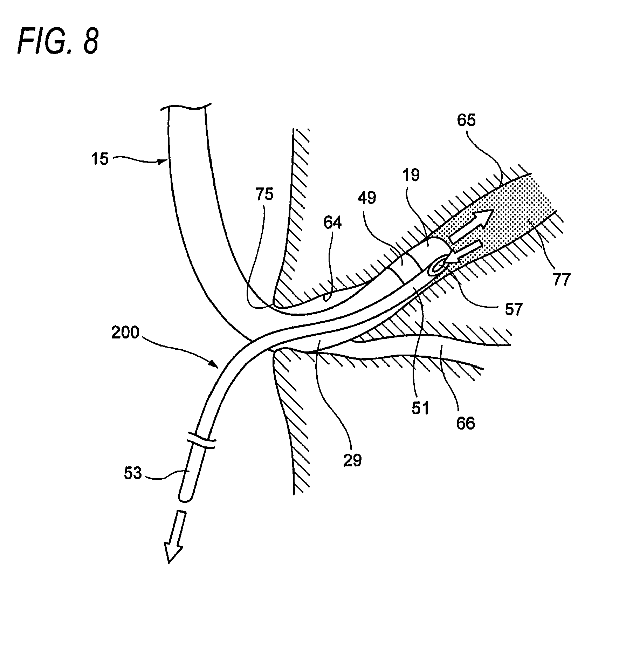

Fig. 7 is a front view of the vicinity of the duodenum and a partly cutaway enlarged view of main part thereof. Fig. 8 is an enlarged view of the endoscope front end portion 19 and the drainage tube 200 inserted into the bile duct 65 at the time of drainage.

In the drainage tube-including endoscope 100, the endoscope front end portion 19 is inserted up to the duodenum 73 located near the liver 67, the stomach 69 and the gallbladder 71. In the duodenum 73, the bile duct 65 and the pancreatic duct 66 are opened at the duodenal papilla 75. Incidentally, the bile duct 65 and the pancreatic duct 66 merge into one common duct 64 connected to the duodenal papilla 75.

First, the curvature portion 29 is bent by operation of the angle knob 31, the endoscope front end portion 19 turned to the duodenal papilla 75 is inserted in the duodenal papilla 75. On this occasion, the front end side tube region 51 is tapered off by the front inclined surface 57, so that insertion resistance can be reduced. As shown in Fig. 8, the endoscope front end portion 19 having passed through the duodenal papilla 75 is disposed in the bile duct 65 via the common duct 64. In the drainage tube 200 fixed to the endoscope front end portion 19, the rear end side tube region 53 is led out to the duodenum 73 from the duodenal papilla 75.

A water feed device is inserted from the forceps insertion portion 41 provided in the connecting portion 37 of the drainage tube-including endoscope 100. Transparent fluid (physiological salt solution) supplied by the water feed device is blown out from the forceps port 43 to the bile duct 65. The blown-out transparent fluid is stirred with the bile, so that a mixed liquid 77 exhibiting a positive pressure in the bile duct 65 is led into the front end side tube region 51 of the drainage tube 200 fixed to the endoscope front end portion 19. The mixed liquid 77 led in from the front end side tube region 51 is discharged from the rear end side tube region 53 to the duodenum 73.

When the bile is replaced with the transparent fluid so that good visibility can be kept, detailed observation is performed by the illumination optical system 21 and the image pickup optical system 23. After completion of the observation, the endoscope front end portion 19 is removed from the bile duct 65. On this occasion, the front end side tube region 51 having high elasticity goes into the duodenal papilla 75 from the interface tip 59 side of the rear inclined surface 61, so that the duodenal papilla 75 is gradually enlarged to make it possible to reduce resistance at the time of removal. On the other hand, the rear end side tube region 53 having low elasticity is deformed flexibly to pass through the duodenal papilla 75 without resistance. Although the aforementioned example shows the case where the drainage tube is inserted into the bile duct 65 and removed from the bile duct 65, the drainage tube can be inserted into the pancreatic duct 66 in the same manner.

Thus, in the drainage tube 200 and the drainage tube-including endoscope 100, because the front end side tube region 51 of the drainage tube 200 has high elasticity in the radial direction, the front end side tube region 51 does not collapse at the time of insertion into the duodenal papilla 75 so that drainage for discharging liquid in the bile duct 65 or pancreatic duct 66 can be performed surely. Moreover, because the elasticity of the rear end side tube region 53 is lower than that of the front end side tube region 51, the rear end side tube region 53 is deformed so easily as not to damage the inside of the body cavity at the time of removal.

Hence, according to the aforementioned drainage tube 200 and the aforementioned drainage tube-including endoscope 100, the drainage tube can be indwelled in the bile duct 65 or pancreatic duct 66 easily without forcing the patient to bear an excessive strain. Moreover, it is not necessary to handle the drainage tube carefully and specially but the drainage tube can be inserted together with the endoscope insertion portion into the bile duct or pancreatic duct to form perfusion easily, so that the field of view can be kept quickly and smoothly.

Thus, the invention is not limited to the aforementioned embodiment, and changes or applications made based on the description in this specification and well-known techniques by those skilled in the art are intended by the invention and included in the scope claimed to be protected.

As described above, the following items are disclosed in this specification.
(1) It is a drainage tube attached along a longitudinal direction of an endoscope insertion portion to a front end portion of the endoscope insertion portion inserted into a bile duct or pancreatic duct, the drainage tube comprising: a front end side tube region which is fixed to an outer circumference of the front end portion of the endoscope insertion portion and which has radial elasticity so as not to collapse at the time of insertion into a duodenal papilla; and a rear end side tube region which is provided so as to be consecutive to the front end side tube region, which has an extension length so as to be separate from the front end portion of the endoscope insertion portion and which has lower radial elasticity than that of the front end side tube region.
   According to this drainage tube, the front end side tube region has high elasticity in the radial direction, so that the front end side tube region does not collapse at the time of insertion into the duodenal papilla. Moreover, drainage for discharging liquid from the bile duct or pancreatic duct can be performed surely. Moreover, since the elasticity of the rear end side tube region is lower than that of the front end side tube region, the rear end side tube region can be deformed so easily as not to damage the inside of the body cavity at the time of removal of the endoscope.
(2) In the drainage tube according to (1), the front end side tube region is formed in such a manner that a front end side of the endoscope insertion portion is cut obliquely with respect to a tube axial direction, and that the cut tip is disposed on an outer circumferential side of the endoscope insertion portion.
   According to this drainage tube, when the front end side tube region is fixed to the front end portion of the endoscope insertion portion, the tube tip is disposed so as to abut on the outer circumference of the endoscope front end portion. The front inclined surface is provided as an inclined surface which gradually comes closer to the outer circumferential surface of the endoscope front end portion as the position goes toward the front in the tube axial direction (toward the endoscope front end side). Thus, the drainage tube is tapered off so that the front end portion of the endoscope insertion portion to which the drainage tube is unitedly fixed can be inserted into the duodenal papilla easily. Moreover, because the front end of the tube can be provided as a large opening, the flow volume of liquid in the tube can be gained.
(3) In the drainage tube according to (1) or (2), the front end side tube region and the rear end side tube region are connected to each other by an interface inclined with respect to the tube axial direction so that a tip of the interface in the front end side tube region is disposed on an outer circumferential side of the endoscope insertion portion.
   According to this drainage tube, when both the endoscope insertion portion and the drainage tube are removed from the bile duct or pancreatic duct, the front end side tube region having high elasticity goes into the duodenal papilla from the interface tip side so that the duodenal papilla can be gradually enlarged to reduce resistance at the time of removal.
(4) It is a drainage tube-including endoscope, in which a drainage tube according to any one of (1) to (3) is detachably fixed to the front end portion of the endoscope insertion portion.
   According to this drainage tube-including endoscope, the drainage tube can be afterward attached to an existing endoscope, and the drainage tube can be exchanged for a new drainage tube or a drainage tube with a different length.
(5) In the drainage tube-including endoscope according to (4), the cut tip of the front end side tube region is disposed in the same position as a front end surface of the endoscope insertion portion in the longitudinal direction of the endoscope insertion portion or in a position backed from the front end surface.
   According to this drainage tube-including endoscope, the drainage tube is prevented from coming into sight so that an observation image having a wide angle of view can be obtained. Moreover, because the tube front end region is backed from the front end surface of the endoscope insertion portion, transparent fluid which has just been supplied from the front end portion of the endoscope insertion portion is not discharged from the front end side tube region immediately so that the supplied transparent fluid can be stirred with the bile surely in front of the front end surface of the endoscope insertion portion.
(6) In the drainage tube-including endoscope according to (4), the cut tip of the front end side tube region is disposed so as to protrude outward in the tube axial direction from a front end surface of the endoscope insertion portion.
   According to this drainage tube-including endoscope, the opening of the drainage tube is prevented from abutting on the inner wall of the body cavity so that the bile can be discharged more smoothly without reduction in the discharged fluid flow volume. Accordingly, perfusion is apt to be formed so that the visual field can be kept quickly for sharp observation of the inside of the bile duct or pancreatic duct.
(7) In the drainage tube-including endoscope according to any one of (4) to (6), the endoscope insertion portion has a curvature portion which can be operated to be curved only in one direction; and the front end side tube region is fixed to a curvature outside of the endoscope insertion portion subjected to the curving operation.
   According to this drainage tube-including endoscope, when the curvature portion is operated to be curved, the drainage tube is disposed on the outside of the curved curvature portion so that the drainage tube neither obstructs the operation of curving the curvature portion nor collapses due to the curvature of the curvature portion.
(8) In the drainage tube-including endoscope according to any one of (4) to (7), the rear end side tube region has a length led out of a body cavity from the position of the front end portion of the endoscope insertion portion inserted into the bile duct or pancreatic duct.
   According to this drainage tube-including endoscope, the base end side of the drainage tube can be led out of the body cavity, and a suction pump, a collection vessel or a syringe can be connected to the end of the base end side of the drainage tube in order to collect the bile.

### Industrial Applicability

According to the invention, a drainage tube can be indwelled in a bile duct or pancreatic duct easily without forcing a patient to bear an excessive strain.

This application is based on Japanese Application (Patent Application 2010-192984) filed on August 30, 2010, and the contents thereof are incorporated here as reference.

**Reference Signs List**

| | |
|---|---|
| 15 | endoscope insertion portion |
| 19 | front end portion |
| 29 | curvature portion |
| 51 | front end side tube region |
| 53 | rear end side tube region |
| 55 | tube tip |
| 59 | interface tip |
| 63 | front end surface |
| 65 | bile duct |
| 66 | pancreatic duct |
| 75 | duodenal papilla |
| 100 | drainage tube-including endoscope |
| 200 | drainage tube |

## Claims

1. A drainage tube attached along a longitudinal direction of an endoscope insertion portion to a front end portion of the endoscope insertion portion inserted into a bile duct or pancreatic duct, the drainage tube comprising:
a front end side tube region which is fixed to an outer circumference of the front end portion of the endoscope insertion portion and which has radial elasticity so as not to collapse at the time of insertion into a duodenal papilla; and
a rear end side tube region which is provided so as to be consecutive to the front end side tube region, which has an extension length so as to be separate from the front end portion of the endoscope insertion portion and which has lower radial elasticity than that of the front end side tube region.

2. The drainage tube according to Claim 1, wherein:
the front end side tube region is formed in such a manner that a front end side of the endoscope insertion portion is cut obliquely with respect to a tube axial direction, and that the cut tip is disposed on an outer circumferential side of the endoscope insertion portion.

3. The drainage tube according to Claim 1 or 2, wherein:
the front end side tube region and the rear end side tube region are connected to each other by an interface inclined with respect to the tube axial direction so that a tip of the interface in the front end side tube region is disposed on an outer circumferential side of the endoscope insertion portion.

4. A drainage tube-including endoscope, in which a drainage tube according to any one of Claims 1 to 3 is detachably fixed to the front end portion of the endoscope insertion portion.

5. The drainage tube-including endoscope according to Claim 4, wherein:
the cut tip of the front end side tube region is disposed in the same position as a front end surface of the endoscope insertion portion in the longitudinal direction of the endoscope insertion portion or in a position backed from the front end surface.

6. The drainage tube-including endoscope according to Claim 4, wherein:
the cut tip of the front end side tube region is disposed so as to protrude outward in the tube axial direction from a front end surface of the endoscope insertion portion.

7. The drainage tube-including endoscope according to any one of Claims 4 to 6, wherein:
the endoscope insertion portion has a curvature portion which can be operated to be curved only in one direction; and
the front end side tube region is fixed to a curvature outside of the endoscope insertion portion subjected to the curving operation.

8. The drainage tube-including endoscope according to any one of Claims 4 to 7, wherein:
the rear end side tube region has a length led out of a body cavity from the position of the front end portion of the endoscope insertion portion inserted into the bile duct or pancreatic duct.
